# EUROPEAN PATENT APPLICATION

(11) **EP 3 576 020 A1**
(43) Date of publication of application: **04.12.2019**
(21) Application number: 18204072.5
(22) Date of filing: 02.11.2018
(51) Int. Cl.: G06N 3/04

(54) **METHODS FOR GENERATING SYNTHETIC TRAINING DATA AND FOR TRAINING DEEP LEARNING ALGORITHMS FOR TUMOR LESION CHARACTERIZATION, METHOD AND SYSTEM FOR TUMOR LESION CHARACTERIZATION, COMPUTER PROGRAM AND ELECTRONICALLY READABLE STORAGE MEDIUM**

(30) Priority: 30.05.2018 EP 18175136; 11.07.2018 EP 18182895
(71) Applicant: Siemens Healthcare GmbH, 91052 Erlangen (DE)
(72) Inventor: Katzmann, Alexander, 91094 Langensendelbach (DE); Kratzke, Lisa, 91054 Erlangen (DE); Mühlberg, Alexander, 90409 Nürnberg (DE); Sühling, Michael, 91052 Erlangen (DE)

(57) **Abstract**

Method for generating synthetic training data for training a deep learning algorithm (1), comprising the steps of:
- training a Generative Adversarial Network (5) to generate synthetic image data (4), wherein the Generative Adversarial Network (5) comprises a generator (6) and a discriminator (7),
- using the generator (6) of the Generative Adversarial Network (5) to generate synthetic image data (4) as the synthetic training data.

## Description

The invention relates to a method and system for tumor biology visualization and assessment, in particular for characterization and/or tumor biology visualization and assessment of colorectal cancer, and/or for lung nodule characterization. Specifically the invention relates to a method and system for lung nodule characterization and/or tumor lesion characterization and/or tumor biology visualization and assessment via machine learning, in particular deep learning. The invention further relates to a computer program and an electronically readable storage medium.

The invention is generally in the field of medical imaging and medical image analysis. Medical imaging can be performed with a variety of imaging modalities such as X-ray, ultra sound, magnetic resonance imaging (MRI), computed tomography (CT), PET, SPECT etc. as are known in the art. The imaging is performed with a respective imaging device, also referred to as "scanner".

Lung nodules are found in about one in a hundred chest CT scans worldwide. Overall, approximately 150,000 lung nodules per year are found in the US only. Most of these nodules are granulomas (inflamed tissue) or hamartomas (benign tumors) that are not explicitly life threatening. Hence, the classification of lung lesion's malignancy is a crucial task in radiology that can guide the differential diagnosis and lead to a patient-tailored treatment decision. When lung cancer is misdiagnosed, the patient may receive improper treatment, treatment too late, or no treatment at all, resulting in significantly lower survival chances. (Semi-)Automatic classification of tumor malignancy could on one hand help to improve patient survival rate and on the other hand reduce costs of public health services. With the recent introduction of coverage and reimbursement for lung cancer screening using low dose CT scanning in the US, the need and potential for computer-assisted decision support for lung nodule characterization increased dramatically.

Colorectal cancer is the second leading cause of cancer related deaths in modern societies, being responsible for more than 50,000 deaths in the U.S. in 2017 alone. More than 50% of patients with colorectal cancer develop liver metastases, which may lead to liver organ failure, additional organ compression and thus significantly reduce patient life time.

In colorectal cancer treatment a patient is scanned with computed tomography (CT) every 2-3 months (depending on tumor stage), to rule out liver and/or lung metastases, being the most common sites for CRC. Such metastases are correlated with significantly reduced patient survival (non-metastatic: 53-92%, depending on stage; metastatic: 11%). Current assessment of liver lesions is done using the Response Evaluation Criterion for Solid Tumors (RECIST). When liver lesions are found, using RECIST, lesion therapy response is assessed by measuring the longest lesion diameter within one tumor slice. After another 2-3 months the lesion is re-assessed with the same measurement. By taking the ratio of both measurements, the lesion response is deduced. Taking RECIST's criteria, a shrinkage of at least 30% indicates a partial therapy response (PR), while at least 20% growth implies a progressive disease (PD). Neither significant growth nor shrinkage are classified as a stable disease (SD). Applying these criteria has one major downside, as it is only applicable retrospectively. Thus, when patients have neither PD- nor PR-status, their medication cannot be adapted as RECIST does not allow direct conclusions on future tumor growth.

As already discussed, characterizing nodules found in CT (Computed Tomography) chest images or tumor lesions found in CT images in general is mainly guided by the radiologist's experience, who uses visual criteria such as spiculation, lobulation or sphericity. Termed "Radiomics", some of these criteria can be described partially by automatically extractable, but hand-crafted, image features whose relation to the quality of nodule characterization remains speculative. Concerning "Radiomics", please also refer to the following articles:
- Aerts, H., et al. (2014). Decoding tumor phenotype by noninvasive imaging using a quantitative radiomics approach. Nature communications, 5, 4006.
- Gillies, R. J., Kinahan, P. E., and Hricak, H. (2015). Radiomics: images are more than pictures, they are data. Radiology, 278(2), 563-577.

A more general, automatic way to represent visual lesion variation would neglect the necessity for feature crafting by hand, where there is no guarantee for hand-crafted features to be suitable for the final classification task. For most domains, current image classification algorithms mainly employ deep convolutional neural networks (DCNNs/Deep Learning) where the identification of appropriate image features is inherently done in the DCNN training process. Since medical data is often rare and not publicly available - due to privacy concerns or ethical aspects, while DCNNs contain a large number of free parameters, they usually do not reach the quality of more classical conservative approaches, e.g. random forests, when the amount of available data is very limited.

It is an object of the invention to address these limitations due to limited data availability, in particular to provide means for high quality tumor lesion characterization despite limited data availability.

This object is achieved by providing methods, systems, computer programs and electronically readable storage mediums according to the independent claims. Preferred embodiments are described in the dependent claims.

In particular, the invention relates to the methods as generally described herein for training artificial intelligence algorithms and/or methods and systems to perform tumor lesion characterization, in particular for lung nodule characterization or for characterization and/or tumor biology visualization and assessment of colorectal cancer. The invention further relates to the methods as generally described herein for generating synthetic training data for training such artificial intelligence algorithms and/or systems to perform tumor lesion characterization. The invention further relates to the methods as generally described herein for tumor lesion characterization. The invention further relates to a system as generally described herein for tumor lesion characterization. The invention further relates to any computer program which performs the steps of the methods of the invention, if the computer program is executed on an computer, and to any electronically readable storage medium, on which any of the computer programs of the invention can be stored.

Specifically, in a first aspect, the invention relates to a method to generate synthetic training data for training a deep learning algorithm, comprising the steps of
- training a Generative Adversarial Network (GAN) to generate synthetic image data, wherein the GAN comprises a generator and a discriminator,
- using the generator of the GAN to generate synthetic image data as the synthetic training data.

Generative adversarial networks (GANs) are a class of artificial intelligence algorithms used in unsupervised machine learning, implemented by a system of two neural networks contesting with each other in a zero-sum game framework. (Please refer to the article by Goodfellow et al. (2014). Generative Adversarial Networks. In: Advances in neural information processing systems (pp. 2672-2680)). This technique can generate images that look at least superficially authentic to human observers, having many realistic characteristics. One network generates candidates and the other evaluates them. Typically, the generative network learns to map from a latent space to a particular data distribution of interest, while the discriminative network discriminates between instances from the true data distribution and candidates produced by the generator. The generative network's training objective is to increase the error rate of the discriminative network (i.e., "fool" the discriminator network by producing novel synthesized instances that appear to have come from the true data distribution). In practice, a known dataset serves as the initial training data for the discriminator. Training the discriminator involves presenting it with samples from the dataset, until it reaches some level of accuracy. Thereafter, samples synthesized by the generator are evaluated by the discriminator. Backpropagation can be applied in both networks so that the generator produces better images, while the discriminator becomes more skilled at identifying synthetic images. The generator can typically comprise a deconvolutional neural network, and the discriminator can comprise a convolutional neural network. In other words, preferably, both the generator and the discriminator are implemented as neural networks, wherein in particular the generator comprises a deconvolutional network and the discriminator comprises a convolutional network, and/or backpropagation is applied to both the generator and the discriminator.

Regarding the deep learning algorithm, it preferably is or comprises a deep convolutional neural network (DCNN) and is, of course, not part of the GAN itself (external to the GAN). The deep learning algorithm generally uses input data to determine output information, wherein the input data comprises image data, in particular medical image data of a subject patient (subject patient image data). The output information may comprise assistive diagnostic information for supporting a diagnosis to be made by a healthcare professional. For example, in the case of tumor lesion characterization, the output information may be a classification of a tumor lesion shown in the subject patient image data, in particular benign/malignant, and/or a survival estimate and/or other assistive information. That is, the deep learning algorithm may be an algorithm for computer aided/assisted diagnosis (CAD). The output information, thus, may only serve as a decision support and not be a diagnosis itself.

In particular, the GAN produces synthetic medical image data to be used as training data for training the deep learning algorithm. Such image data, also relating to the known dataset used for training the discriminator, may be associated with a medical imaging modality, for example magnetic resonance imaging, ultrasound imaging and/or x-ray. Preferably, the image data used and synthesized is associated with x-ray imaging, in particular computed tomography (CT).

In summary, the invention provides training data synthetization, preferably augmentation, using semi-synthetic image data: For training with few data, we propose Generative Adversarial Networks (GANs) to systematically create synthetic data based, in particular, on semi-supervised learning. A GAN generally consists of two parts: A) a generator, creating synthetic images from a specified amount of random numbers, B) a discriminator, determining whether an image is original or synthetic. In a null-sum-game the GAN is trained to create realistic images by its generator part. Combined with a pre-trained, even weak neural network in a bootstrapping process, the network is used to determine the realism of the GAN-generated synthetic data to subsequently improve the synthetic data quality. After training the GAN, the generator is used to create additional, realistic, synthetic training data, not contained in the original dataset (known dataset) resulting in a realistic enrichment of the data pool. In preferred embodiments, therefore, a known dataset used as training data for the discriminator is enriched by the synthetic image data, wherein a combination of data from the known dataset and the synthetic image data forms the training data for the deep learning algorithm.

It may also be provided that the deep learning algorithm is or comprises a classifier sub-algorithm outputting, as output information, a classification by associating at least one final target classification level to input data, and multiple Generative Adversarial Networks are trained and used, one for each final target classification level. For example, different GANs may be trained for benign and malignant tumor lesions to further improve the quality of the generated synthetic image data by focussing on a certain class of input data and the variability of the properties of this class in the image.

When multiple GANs are trained, one per final target classification label (e.g. two GANs for benign/malignant nodule creation), the training set is augmented with synthetic but realistic data. This technique allows to prevent the often occurring problem that a network does not generalize when the ratio of free parameters to available data sets is too large. Our realistic synthetic data enrichment based on Auto-discriminative tumor lesion Characterization using Deep Learning (ACDL) also allows increasing the complexity, i.e. the amount of free parameters, of the network, resulting in a superior classification performance.

The current invention, in summary, allows deep learning with few data through realistic training data augmentation. Although different image based augmentation techniques exist to improve performance on smaller pools of data (e.g. affine image transforms), the semantic of the resulting images with respect to the classification goal remains unsure. Affine image transforms may highly interfere with biological information and therefore destroy diagnostically relevant image information, resulting in an unrealistic dataset augmentation and a significantly lower generalization performance. Using realistic, semi-synthetic data generated with GANs allows semantic data augmentation and therefore enables automatic disease classification through deep learning effectively with very few data. This constitutes a competitive advantage, as state-of-the-art computer aided diagnosis often employs several hundreds to thousands of images, often collected over multiple studies with various study designs, for training machine learning algorithms. In contrast, the inventive method using GANs to create synthetic image data reaches equal or superior performance while preserving the specific study environment requiring only a fraction of the data.

The GAN, in particular at least its discriminator, can, for example, be trained with training data comprising image data obtained from patient image data. This can be image data from a subject patient whose tumor lesions are to be characterized. It can also be image data from other patients.

Preferably, as already explained, a known dataset used as training data for the discriminator is enriched by the synthetic image data, wherein a combination of data from the known dataset and the synthetic image data forms the training data for the deep learning algorithm. The invention thus allows augmentation of smaller training data sets, in particular medical patient image data, by adding synthetic image data generated by the GAN.

As will be discussed in more detail in the following, it is generally preferred in the current invention to use a deep learning algorithm comprising an autoencoder sub-algorithm to generate a sparse representation of input data, in particular subject patient image data, followed by the application of a classifier sub-algorithm to derive the output information from the sparse representation. In other words, the deep learning algorithm may comprise a first set of layers acting as an autoencoder and a second set of layers acting as a classifier.

In such an environment, it may be expedient to use feedback from training the autoencoder sub-algorithm in further training of the GAN to further improve the quality of the output data of both these instances. That is, it is conceivable to, when a sparse representation of image data is used in the deep learning algorithm, use feedback from training an autoencoder sub-algorithm generating the sparse representation for training the Generative Adversarial Network.

In a second aspect, the invention also concerns a method for training a deep learning algorithm to perform a tumor lesion characterization, in particular a lung nodule characterization or for characterization and/or tumor biology visualization and assessment of colorectal cancer, on image data of a region of the anatomy of a subject patient, comprising the step of training the deep learning algorithm with training data comprising synthetic image data. In particular, the synthetic image data as training data was generated in a method according to the first aspect of the invention. Preferably, the training data also comprises image data obtained from patient image data. In this manner, a small amount of available training data in the form of acquired image data can be enriched/augmented by realistic synthetic data, resulting in a better training and higher quality of output information.

All features and remarks regarding the method to generate synthetic training data also apply here. In particular, the deep learning algorithm may use input data, comprising subject patient image data containing the tumor lesion, to derive output information as described above. Using training data also comprising synthetic image data, in particular synthetic samples added to samples having actually acquired image data, augments the basis for training in a system having many free parameters, as is the case in tumor lesion characterization.

In preferred embodiments, the training step further comprises Adaptive Sample Weighting (ASW). The invention may thus provide training efficacy enhancement through sample difficulty adaption with Adaptive Sample Weighting (ASW). Adaptive Sample Weighting is a novel technique for training neural networks with randomized importance sampling. At its core, ASW is a nested process which presents specific samples to a neural network which are particularly chosen to maximize the predictive performance. The difficulty of predicting cases, measured - in terms of neural networks - as their sample loss, gets transformed into a sampling probability which is proportional to the complexity of the prediction task. To handle occurring oscillation effects - as a naive application of this method leads to the loss of network's prior knowledge - the training is additionally stabilized by the iterative transformation of the sampling probability to a logarithmic equal distribution. As will be discussed below, ASW may advantageously be used when training an autoencoder sub-algorithm for generating a sparse representation.

Using ASW, it is possible to have a hard-to-detect disease emphasis for automatic classification tasks. It is possible to emphasize sub-cohorts of training data whose inherent patterns are hard to automatically detect, e.g. by DCCNs, due to underrepresentation. As ASW is a nested in-training process, each detectable class gets equally distributed within the used neural network, no matter how many samples exist to represent it. This in turn leads to superior predictive performance compared to non-adaptively weighted approaches. Implementing ASW as a nested process ensures the network to always learn on the most instructive training data subset.

ASW combined with generation of synthetic training data using GAN(s) advantageously allows "training-on-the-fly": As mentioned, the deep learning algorithm is capable of training with very few data. This enables on-the-fly-training, meaning that our algorithm can be trained immediately with data acquired in the course of visiting a clinical site, provided that data exists in any form of (X,Y)-tuples, representing image data and target output (output information). Training with few data is enabled by a) semi-synthetic data generation, and b) training efficacy enhancement through sample difficulty adaption.

In especially preferred embodiments, the deep learning algorithm comprises an autoencoder sub-algorithm applied in a first stage to yield a sparse representation of the image data, in particular the image data of the tumor lesion, and a classifier sub-algorithm determining an output information from the sparse representation, in particular associating a final target classification level with the sparse representation. In the case of neural networks, the deep learning algorithm thus comprises a first set of layers acting as an autoencoder and a second set of layers acting as a classifier to derive the output information from the sparse representation. Preferably, the sparse representation may comprise between 10 and 200 parameters, preferably 15 to 25, for example 20, parameters. Using an autoencoder is, again, very advantageous when having few training data, but many free parameters, which may lead to "overfitting" when training the deep learning algorithm. However, by pretraining an autoencoder, as further discussed below, in particular having very few neurons, a bottleneck is formed within the network, enforcing a very general dimensionality reduction.

Sparse factor representation, for example, allows differential diagnosis. Sparse representations allow to differentiate a variety of diseases, as they cover the whole variability of tumor images contained in the training data in only a few values. These sparse representations further allow to be used for a wide range of classification tasks, for example in multi-target characterization.

Embedding, for example, nodule appearance in a sparse representation as presented above does not only allow to predict one specific target, but furthermore enables the full coverage of all lesions' variability contained in training data. This allows to predict a variety of target variables (e.g. tumor malignancy, expected growth, probability for lymph node involvement or metastases, progression free survival, overall survival, ...) utilizing the same underlying factor representation. In addition, having a known target variable as output information, the proposed system can be used to improve this sparse characterization to be more specific, leading to a significantly enhanced classification performance.

When utilizing sparse factor representations for giving an early assessment, an estimate on future tumor growth with an AUC of 0.814, compared to 0.698 with RECIST, is allowed. In the example of using sparse factor representation for liver lesions with deep convolutional autoencoders and utilizing the Adaptive Sample Weight (ASW) proposed herein, it is possible to create sparse semantic representations of liver lesions and their respective growth pattern. Therefore, when having at least two time points, the baseline and one follow-up image are given as input to our system, which in turn provides a sparse factor representation. This sparse factor representation can then be utilized for future estimates. Furthermore, our system is able to cope with baseline images only while reaching comparable performance. The sparse factor representation remains quite general, thus it may be not target specific (that is, not output information-specific) and allows the further prediction of a multitude of target variables as output information.

As explained, the classifier sub-algorithm uses the sparse representation to generate output information. This, for example, allows fully-automatic lesion growth prediction. The sparse factor representation serves as an input to a second stage. This second stage is responsible for the target specific estimation. Generally, this second stage is exchangeable and thus can be used to predict a multitude of target variables. Also the second stage can serve as a target specific guide for the first stage, thus further enhancing the sparse factor representation to reach higher target-specific specificity and sensitivity. Our first prototypical implementation performed significantly better than RECIST in predicting future tumor growth. In our analysis we also showed that estimates provided using fully-volumetric tumor-assessment were not more accurate than RECIST-based predictions, thus our system also outperforms volumetric assessment. This is in particular interesting, as our system only requires a single slice of a liver lesion for assessment, while volumetric assessment requires a fully-volumetric segmentation of lesions.

Another example is automatic prediction of one-year-survival rates. A first prototype of the deep learning algorithm was able to predict one-year-survival better than RECIST-based assessment alone. While these results are not final, they show that the system is capable of predicting multiple target variables using the same sparse factor representation.

As already indicated, in a preferred embodiment, the training step comprises
- training the autoencoder sub-algorithm comprising reconstructing image data from the sparse representation by a reconstruction sub-algorithm and comparing the reconstructed image data to the training data the sparse representation relates to,
- replacing the reconstruction sub-algorithm by the classifier sub-algorithm and training of the deep learning algorithm using the training data.

That is, in a preferred embodiment, the autoencoder sub-algorithm may be pre-trained, in this manner preferably creating a "bottleneck" and forcing a dimensionality reduction, as already explained above. Accordingly, training may take place in two sub-steps. First, the autoencoder is trained to create sparse representations, second, (at least) the classifier appended to the autoencoder sparse representation layer is trained. In an example, the network architecture of the autoencoder sub-algorithm may comprise six to eight layers, to which, for the first training substep, another six to eight layers may be added as a reconstruction sub-algorithm. In other words, the autoencoder sub-algorithm performs a convolution yielding the sparse representation, while the reconstruction sub-algorithm deconvolutes this sparse representation and yields reconstructed image data, which can be compared to the original training data of the sample from which the sparse representation was derived. In the second sub-step, the layers of the reconstruction sub-algorithm are removed and replaced by layers of a classifier sub-algorithm, which, for example, may comprise two new neural layers. The deep learning algorithm completed in this manner is then trained using the training data, wherein, in a first embodiment, it is possible to keep the autoencoder layers fixed. In other embodiments, however, it may also be advantageous to use training results of the classifier sub-algorithm in a further training of the autoencoder sub-algorithm, in particular for modifying the choice of parameters of the sparse representation. In other words, the choice of parameters in the sparse representation can be optimised regarding a specific output information, while, in other embodiments, it may also be possible to keep these layers of the autoencoder sub-algorithm fixed.

In an especially preferred embodiment, adaptive sample weighing is used to adapt weights depending on comparison results of comparing the reconstructed image data to the training data for each sample of the training data. For example, the sampling probability may be multiplied with the norm of the error gradient for each sample, as huge benefits in training and test set performance are encountered.

In many cases, for example in the already discussed case of classification, the data distribution may be highly imbalanced regarding the different classifications. Using ASW, training may focus on underrepresented cases/final classification levels, equalising the distribution. In other words, training may be focussed on samples which are rare and thus erroneously handled by the autoencoder sub-algorithm.

In an embodiment, multiple, exchangeable classifier sub-algorithms determining different output information are used, wherein in particular the autoencoder sub-algorithm is kept fixed independent of any output information. In particular in a case where the autoencoder sub-algorithm is not changed depending on the output information to be determined, that is, a none-output-information-specific autoencoder sub-algorithm is used, multiple, exchangeable classifier sub-algorithms may be used to determine different output information from the same sparse representation. In other words, the sparse representation can prospectively be used to predict a variety of target variables, that is, output information. In experiments regarding liver lesions due to colorectal cancer, first experiments showed prediction of one-year-survival to be superior to RECIST.

It should be noted at this point that dividing the deep learning algorithm into an autoencoder sub-algorithm and a classifier sub-algorithm and training these two in separate sub-steps is also advantageous independent of using GANs to generate synthetic training data. An advantageous method for training a deep learning algorithm to perform a tumor lesion characterisation, comprising the step of training the deep learning algorithm with training data, wherein the deep learning algorithm comprises an autoencoder sub-algorithm applied in a first stage to yield a sparse representation of the image data, in particular the image data of the tumor lesion, and a classifier sub-algorithm determining an output information from the sparse representation, in particular associating a final target classification level with the sparse representation, is conceivable, wherein the training step comprises
- training the autoencoder sub-algorithm comprising reconstructing image data from the sparse representation by a reconstruction sub-algorithm and comparing the reconstructed image data to the training data the sparse representation relates to,
- replacing the reconstruction sub-algorithm by the classifier sub-algorithm and training of the deep learning algorithm using the training data.

Of course, all features and remarks relating to the use of a pre-trained autoencoder also apply to this conceivable method.

In a further advantageous embodiment of the method according to the second aspect of the invention, at least a part of the training data samples of the training data comprises additional non-image input data additionally used for training. In particular, such additional non-image input data may comprise tumor biology information and/or tumor therapy information and/or laboratory values and/or subject patient demographics information. In this way, additional information which may or may not be available may be used to further compensate for only having a few samples in the training data.

This can also be termed "multiple-input data fusion". The proposed method is capable of extracting information from a variety of input data sources. It can, for example, thus integrate information from:

### a) Input images

The system can use 1 to n timepoints of CT imaging data for prediction.

### b) Tumor biology

The system can take additional input, such as location, genetics, e.g. K-RAS, N-RAS, B-RAF, and histology.

### c) Tumor therapy

The deep learning algorithm can be trained for particularly classifying lesions under specific therapies. Furthermore, it can handle the therapy itself as an input parameter.

### d) Laboratory values

Laboratory values can serve as an additional input. They thus improve the classification performance additionally.

### e) Patient Demographics

Additionally, the algorithm can work with demographic information, such as age and gender of the patient.

In a third aspect, the invention relates to a method for tumor lesion characterization, in particular for lung nodule characterization or for characterization and/or tumor biology visualization and assessment of colorectal cancer, comprising the steps of:
- receiving subject patient image data of a region of the anatomy of subject patient,
- analysing said subject patient image data using a trained deep learning algorithm,
- determining an output information regarding a tumor lesion based on the step of analysing,
wherein said trained deep learning algorithm has been trained with training data comprising synthetic image data in accordance with a method according to the second aspect of the invention.

The invention, in this third aspect, further relates to a system for tumor lesion characterization, comprising
- an interface to receive subject patient image data of a region of the anatomy of subject patient,
- an analysing unit for analysing said subject patient image data using a trained deep learning algorithm, wherein said trained deep learning algorithm has been trained with training data comprising synthetic image data in accordance with any of the methods according to the second aspect of the invention,
- a determining unit for determining an output information regarding a tumor lesion based on the step of analysing,
- an interface for outputting said determined output information.

The system may be implemented on a computer, a workstation, in a network of computers or the like. It can be provided as part of an imaging scanner and can be integrated into the scanner's control system. It can also be implemented in a cloud-based solution.

All remarks and features discussed relating to the first and second aspects may also be applied to the method and system of the first aspect. That is, the third aspect relates to the application of the trained deep learning algorithm to obtain output information regarding the tumor lesion.

The output information regarding a tumor lesion can be a target variable output of the deep learning algorithm. In particular, the output information regarding a tumor lesion can be a classification information of the tumor lesion or of a patient disease state (e.g. benign/malignant, tumor stage classification, treatment susceptibility, etc.) or a quantitative parameter (e.g. predicted overall survival rate, five year survival rate, etc.), as already discussed above. Output information regarding a lung nodule may also be a classification information of the lung nodule (e.g. benign/malignant, tumor stage classification, treatment susceptibility, etc.) or a quantitative parameter (e.g. predicted overall survival rate, etc.).

In the application of lung nodule characterization, an Auto-discriminative nodule Characterization using Deep Learning (ACDL) results. Nodule characterization is a crucial task, as it guides further treatment steps as well as differential diagnosis. As deep learning determines discriminative features without the need for hand-crafted design or specification, it can perform significantly better than classical approaches, assuming the data pool used to train the deep learning algorithm, in particular a DCCN, to be large enough. The system preferably employs a combination of augmentation techniques, training improvements and deep convolutional sparse autoencoders (as explained above) to efficiently reduce the needed data pool size to only few samples with great generalization performance.

In particular, early assessment via small-nodule-characterization may be enabled. The proposed approach can be used to characterize smaller lesions with <10mm in diameter, when usually no biopsy is done because of interventional risks. Thus, the method and system according to the third aspect allow giving an early assessment, resulting in better survival chances, as therapy could be initiated in an early tumor stage. As, to the best of our knowledge, there is currently no solution assessing small lung lesions, this in turn results in a competitive advantage.

In general tumor lesion characterization, or in particular when assessing liver lesions due to colorectal cancer, early assessment with lesion growth estimate is possible. Using the proposed deep learning algorithm and system it is possible to give an early assessment of future lesion growth. Thus, the system allows early therapy adaption with practical implications for patient treatment and therefore possibly patient survival.

Of course, other advantages already discussed also apply.

In a preferred embodiment of the method (and system) according to the third aspect, an output information-specific visualization information regarding input regions predictive for specific outcome estimates is determined and the visualization information is output with the output information. To achieve this, guided backpropagation from the output space of the output information to the input space of the subject patient image data may be used. In other words, a so-called saliency map may be determined and used to visualize the reasoning for the decisions of the deep learning algorithm. More information on saliency maps may, for example, be taken from the following articles:
- Karen Simonyan et a. (2013). Deep Inside Convolutional Networks: Visualising Image Classification Models and Saliency Maps. arXiv preprint arXiv:1312.6034.
- Springenberg, J.T. et al. (2015). Striving for Simplicity: The All Convolutional Net. arXiv preprint arXiv:1412.6806.

It is noted that this embodiment is also advantageous independently from using synthetic training data. Thus, a method and system dor tumor lesion characterization and visualization are conceivable. The method would comprise the following steps:
- receiving subject patient image data of a region of the anatomy of subject patient,
- analysing said subject patient image data using a trained deep learning algorithm,
- determining an output information regarding a tumor lesion based on the step of analysing,
- determining an output information-specific visualization information regarding input regions predictive for specific outcome estimates, and
- outputting the visualization information with the output information.

A system would comprise:
- an interface to receive subject patient image data of a region of the anatomy of subject patient,
- an analysing unit for analysing said subject patient image data using a trained deep learning algorithm,
- a determining unit for determining an output information regarding a tumor lesion based on the step of analysing and an output information-specific visualization information regarding input regions predictive for specific outcome estimates,
- an interface for outputting said determined output information and visualization information.

Of course, a combination of using synthetic image data as training data and/or using a sparse representation, in particular applying ASW, with this visualization approach provides additional synergetic advantages, such that all remarks relating to these aspects may still be applied to such a method or system. Generally, the aspect of visualization will be explained in the following in more detail.

A major difficulty when using deep learning for medical image classification arises from the non-interpretability. Neural networks alone are intangible, intransparent and incomprehensible. This applies particularly for end users without deeper technical knowledge, but nevertheless stays true for computer scientists with a well-substantiated knowledge base in machine learning and neural networks, as neural networks apply an intuitively nearly unmanageable amount of nonlinear functions and embeddings. Therefore, it is advantageous to visualize which factors/image regions are responsible for the network's prediction. Thus, a visualisation of indicative regions, for example based on the algorithm by Simonyan (2013) is proposed. The visualization may be created using guided backpropagation of the network activation from the output layer back to the network's input. This allows to visualize regions which are indicative for specific output information, e.g. tumor growth.

In other words, assessment reasoning, in particular also via multi-target saliency maps, may be provided, which is particularly advantageous in a healthcare environment. Understandably, practitioners in healthcare are reserved with fully-automatic estimates on future progresses of disease. It is therefore of high clinical, as well as of high business-value to reason why an algorithm chooses a specific estimate. A novel visualization component based on saliency maps is proposed, which enables target-specific visualization of input regions predictive for specific outcome estimates, e.g. future lesion growth or one-year-survival. This is done via guided propagation from output to input space. Using this approach, it is also possible to visualize combinations, as well as their differences. Thus, the method and system allow the data-driven identification of very specific characteristics in the input region, like image structures and laboratory values (if using additional input data), which, e.g., are predictive for lesion growth but are not predictive for patient survival. For input space visualization, a heat-map-based overlay on the original tumor image is proposed, indicating decision-relevant input regions.

With saliency map visualization, the method and system are capable of reasoning why it decides to estimate, for example, growth or non-growth. It does so by visualizing decision-relevant input regions in form of a heat-map overlay on the original image. The reasoning mechanism can be applied to a multitude of input variables and is not specific to images but can also be applied to additional input data/information, e.g. clinical or demographic data.

Further details and advantages of the current invention can be taken from the following description of preferred embodiments in conjunction with the drawings, in which:
- Fig. 1: is a schematic drawing depicting the methods and system of the invention,
- Fig. 2: shows synthetic image data created by a method of the invention which can be used as synthetic training data in further methods of the invention',
- Fig. 3: is a receiver-operating characteristic for lung lesion malignancy classification with the proposed system,
- Fig. 4: shows an example of a visualization of a saliency map, and
- Fig. 5: is a schematic drawing showing a system according to the invention.

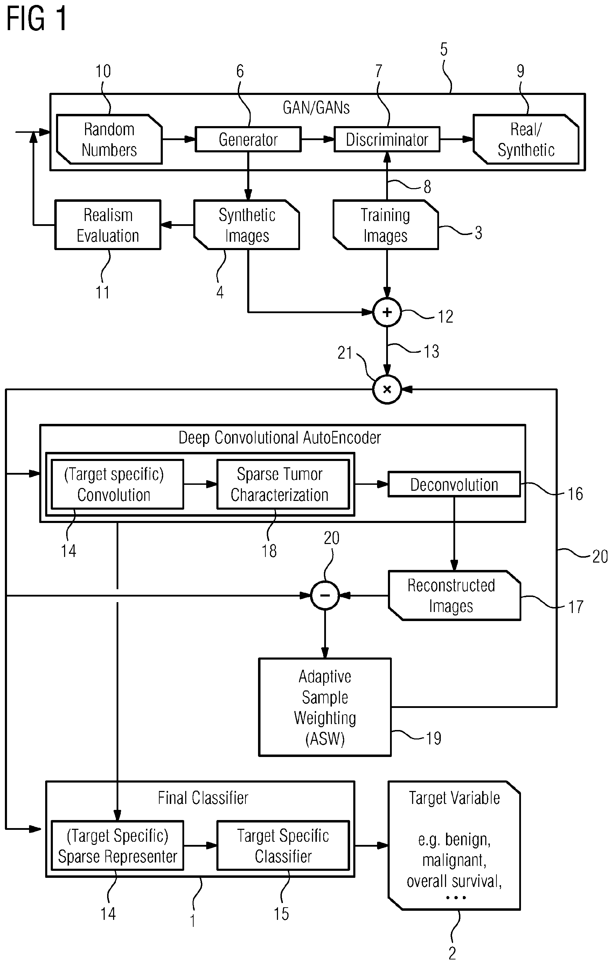

Fig. 1 is a schematic drawing allowing to explain the methods and systems of the current invention. The object of the method and systems explained below is to train and finally use a deep learning algorithm 1 to derive output information 2 (target variables) from input data comprising subject patient image data showing at least one tumor lesion in an area of the anatomy of the subject patient. The output information 2 describes the tumor lesion and/or its implications, for example a classification of the tumor lesion to be characterized by the deep learning algorithm 1, in particular if it is benign or malignant, or probabilities therefore, respectively. Another example of output information 2 is a classification level or value describing the expected survival of the subject patient.

While it can generally be said that the deep learning algorithm 1 can be trained to characterize various different tumor lesions, the current invention can be especially advantageously applied to lung nodules and/or liver lesions, in particular in conjunction with colorectal cancer, as tumor lesions.

In such medical scenarios, the amount of actually acquired patient image data 3, from which image data as training data for the deep learning algorithm may be derived, is very small, in particular in comparison with the number of free parameters in the deep learning algorithm 1, which, in these embodiments, may in particular be a deep convolution neural network (DCNN).

Due to the sparsity of actually acquired patient image data 3 as training data, the method according to the first aspect of the invention concerns augmentation of the training data, i.e. adding synthetic image data 4 to the already available image data taken from patient image data 3 (known dataset), wherein it is noted that the patient image data 3 may have been taken from the subject patient, but may also have been acquired from other patients. In the principle application cases discussed above - lung nodules and liver lesions in conjunction with colorectal cancer -, the patient image data 3 may in particular be CT image data, meaning that synthetic image data 4 to be added to the training data should also be associated with CT imaging, in particular look like CT images.

To synthesize a synthetic image data 4, a Generative Adversarial Network (GAN) 5 is used, where it is preferable to use multiple GANs 5 if different classifications (final classification levels) may result as output information 2, such that one GAN 5 is used for each classification, for example benign and malignant.

As is in principle known from the state of the art, a GAN 5 comprises a generator 6 and a discriminator 7, in this case both implemented as neural networks. While the generator comprises a deconvolutional network, a discriminator comprises a convolutional network. The discriminator 7, according to arrow 8, is trained by using the real patient image data 3 such that it may decide whether an image provided is real or synthetic, see decision data 9 in fig. 1. In a "null-sum game", the generator 6 now tries to "fool" the discriminator 7 by creating synthetic images based on random numbers 10. In particular, backpropagation is applied to both the generator 6 and the discriminator 7. For synthetic image data 4, a realism evaluation 11 is applied based on the decision data 9.

Once the GAN 5 has been sufficiently trained, it may generate synthetic image data 4 to be added to the patient image data 3, see step 12, to obtain training data to be altogether used for training the deep learning algorithm 1 in a method according to the second aspect of the invention.

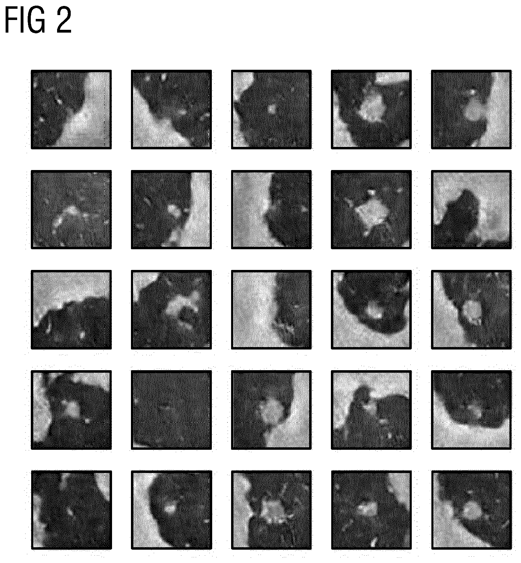

Examples for synthetic image data 4 created by a method according to the first aspect of the invention are shown in fig. 2 for the example of lung nodules.

Returning to fig. 1, a method according to the second aspect of the invention, that is, for training the deep learning algorithm 1, is illustrated in the lower part of fig. 1. The training data being a combination of image data from the patient image data 3 and synthetic image data 4 is used, according to arrow 13. It is noted that usually the training data comprises samples, wherein each sample comprises input data for the deep learning algorithm 1 as well as the ground truth information, in particular relating to or being known output information 2. In the embodiments described here, the input data may not only comprise subject patient image data, but also additional non-image input data, for example tumor biology information, tumor therapy information, laboratory values and/or subject patient demographics information.

As can be seen in fig. 1, the deep learning algorithm 1 comprises two sub-algorithms, namely an autoencoder sub-algorithm 14 and a classifier sub-algorithm 15, each in particular comprising multiple layers of the deep convolutional neural network. The autoencoder sub-algorithm 14 creates a sparse representation 18 of the input data by convolution, in this manner creating a "bottleneck", essentially reducing the dimensionality of the problem. In this example, the sparse representation 18 comprises twenty parameters.

This sparse representation 18 may or may not be target-specific, that is, output information specific, as explained above.

In any case, training of the deep learning algorithm 1 takes place in two sub-steps. In a first sub-step, the autoencoder sub-algorithm 14 is pre-trained, that is, the layers of the classifier sub-algorithm 15 are removed and replaced by a reconstruction sub-algorithm 16 which creates reconstructed image data 17 from sparse representations 18 determined by the autoencoder sub-algorithm 14. The training in this first sub-step is not specific for the output information 2. However, since the training data usually is not equally distributed over, for example, multiple possible classifications, Adaptive Sample Weighting (ASW) 19 is employed regarding the comparison of reconstructed image data 17 and the original image data of the sample used to create the respective sparse representation 18, wherein the comparison is indicated by step 20 in fig. 1. Using ASW, where cases which lead to sparsification errors can be emphasised by adjusting the sampling probability, see arrow 20 and step 21.

Once the autoencoder sub-algorithm 14 has been pre-trained, the deconvolutional layers of the reconstruction sub-algorithm 16 are removed and the layers of the output information-specific classifier sub-algorithm 15 are added to create the full deep learning algorithm 1. Note that, since the sparse representation 18 is not (yet) output information-specific, multiple classifier sub-algorithms 15 may be used providing different output information 2. It is, however, noted that results of the now following second sub-step may, in embodiments, of course also be used to adapt the sparse representation 18 to specific output information 2.

In any case, in the second sub-step of the training step, the deep learning algorithm 1 itself is now trained according to the training data.

After adequate training of the deep learning algorithm 1, it may be used in a method according to the third aspect of the invention and thus a system according to the third aspect of the invention, respectively, to provide tumor lesion characterization.

The proposed methods and systems were, for example, tested on lung lesion images and resulted in great performance boosts for lung lesion malignancy classification compared to Radiomics or comparable deep learning approaches. The methods and systems reach results easily comparable to the state of the art with much simpler network architecture and/or less training samples, both resulting in advantages when used for fewer-case-studies in clinical applications. First experiments also show transferability of the same methods to different application tasks.

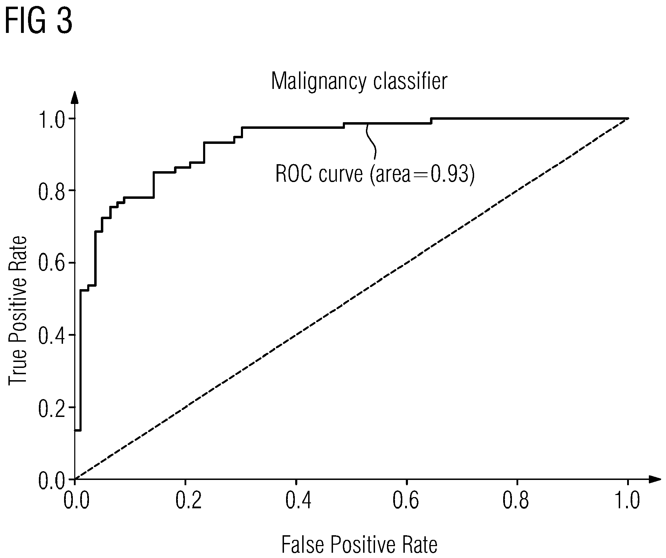

Fig. 3 shows a Receiver-Operating-Characteristic (ROC) for lung lesion malignancy classification with a system according to the invention. The x-axis shows the false positive rate / 1 - specifity, the y-axis shows a true positive rate / sensitivity. As can be seen, superb classification performance can be achieved.

The current invention further allows to calculate saliency maps providing a reasoning for the decisions of the deep learning algorithm 1. For example, an adapted algorithm as proposed by K. Simonyan in the above-cited paper may be used.

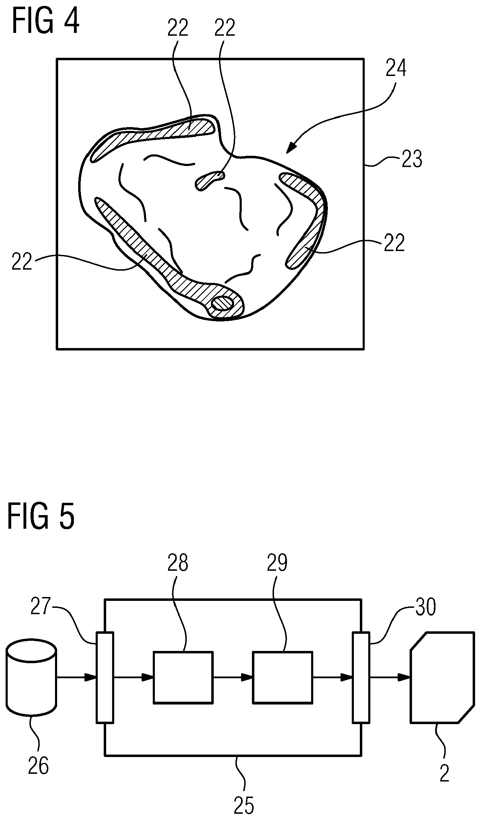

Fig. 4 shows an example for such an output information-specific visualization regarding input regions 22 predictive for specific outcome estimates in a heat-map like overlay, such that an overlaid image 23 showing both the segmented tumor lesion 24 and the image regions 22 by respective coloring is provided.

In the example of fig. 4, a single-slice segmented liver lesion is shown as tumor lesion 24 having an overlay visualization of image regions 22 predictive for future tumor growth. As can be seen, future growth is - expectedly - likely due to marginalized contrast agent absorption. However, inner structuring is predictive in this case, too.

Fig. 5 finally shows a schematic drawing of principal components of a system 25 according to the invention.

The system 25 receives subject patient image data 26 via an interface 27. An analyzing unit 28 analyzes the subject patient image data 26 using the deep learning algorithm 1. A determining unit 29 determines the output information 2, which is output using an interface 30.

To the best of our knowledge there is currently no alternative solution combining the advantages described herein. Hand-crafted radiomics features, as described herein, may also be usable for multiple targets. However, with hand-crafted features only a subset is used for the classification. As the concrete choice on the used subset highly depends on the classification task, this prevents the use as a general lesion characterization. Furthermore, with hand-crafted features no feasible generalization performance can be expected, as they are highly prone to variation through scanner parameters or irrelevant biological interference. Especially there exists no system providing an early assessment for liver lesions on future growth or survival, whether using or not using a sparse characterization. Radiomics can be seen as an alternative technical solution for the problem, but in our experiments they did not perform better than RECIST or volume and, thus, show no inventive benefits. Our system, however, performed significantly better than RECIST or volume based assessment.

Although the present invention has been described in detail with reference to the preferred embodiment, the present invention is not limited by the disclosed examples from which the skilled person is able to derive other variations without departing from the scope of the invention.

## Claims

1. Method for generating synthetic training data for training a deep learning algorithm (1), comprising the steps of:
- training a Generative Adversarial Network (5) to generate synthetic image data (4), wherein the Generative Adversarial Network (5) comprises a generator (6) and a discriminator (7),
- using the generator (6) of the Generative Adversarial Network (5) to generate synthetic image data (4) as the synthetic training data.

2. Method according to claim 1, **characterized in that** both the generator (6) and the discriminator (7) are implemented as neural networks, wherein in particular the generator (6) comprises a deconvolutional network and the discriminator (7) comprises a convolutional network, and/or backpropagation is applied to both the generator (6) and the discriminator (7).

3. Method according to claim 1 or 2, **characterized in that** the Generative Adversarial Network (5) is trained with training data comprising image data obtained from patient image data (3) of the subject patient and/or other patients.

4. Method according to one of the preceding claims, **characterized in that** a known dataset (3) used as training data for the discriminator (7) is enriched by the synthetic image data (4), wherein a combination of data from the known dataset (3) and the synthetic image data (4) forms the training data for the deep learning algorithm (1).

5. Method according to one of the preceding claims, **characterized in that** the deep learning algorithm (1) is or comprises a classifier sub-algorithm (15) outputting, as output information (2), a classification by associating at least one final target classification level to input data and multiple Generative Adversarial Networks (5) are trained and used, one for each final target classification level.

6. Method according to one of the preceding claims, **characterized in that** a sparse representation (18) of image data is used in the deep learning algorithm (1), wherein feedback from training an autoencoder sub-algorithm (14) generating the sparse representation (18) is used for training the Generative Adversarial Network (5).

7. Method for training a deep learning algorithm (1) to perform a tumor lesion characterization, in particular a lung nodule characterization or for characterization and/or tumor biology visualization and assessment of colorectal cancer, on image data (26) of a region of the anatomy of a subject patient, comprising the step of training the deep learning algorithm (1) with training data comprising synthetic image data (4).

8. Method according to claim 7, **characterized in that** the synthetic image data (4) as training data was generated in a method according to any of the claims 1 to 6 and/or that the training data also comprises image data obtained from patient image data (3).

9. Method according to claim 7 or 8, **characterized in that** the training step further comprises Adaptive Sample Weighting (19) .

10. Method according to any of the claim 7 to 9, **characterized in that** the deep learning algorithm (1) comprises an autoencoder sub-algorithm (14) applied in a first stage to yield a sparse representation (18) of the image data, in particular the image data of the tumor lesion, and a classifier sub-algorithm (15) determining an output information (2) from the sparse representation (18), in particular associating a final target classification level with the sparse representation (18).

11. Method according to claim 10, **characterized in that** the training step comprises:
- training the autoencoder sub-algorithm (14) comprising reconstructing image data (17) from the sparse representation (18) by a reconstruction sub-algorithm (16) and comparing the reconstructed image data (17) to the training data the sparse representation (18) relates to,
- replacing the reconstruction sub-algorithm (16) by the classifier sub-algorithm (15) and training of the deep learning algorithm (1) using the training data.

12. Method according to claim 11, **characterized in that** training results of the classifier sub-algorithm (15) are used in a further training of the autoencoder sub-algorithm (14), in particular for modifying parameters of the sparse representation (18).

13. Method according to claim 11 or 12, **characterized in that** Adaptive Sample Weighting (19) is used to adapt weights depending on comparison results of comparing the reconstructed image data (17) to the training data for each sample of the training data.

14. Method according any of the claims 10 to 13, **characterized in that** multiple, exchangeable classifier sub-algorithms (15) determining different output information (2) are used, wherein in particular the autoencoder sub-algorithm (14) is kept fixed independent of any output information (2).

15. Method according to any of the claims 7 to 14, **characterized in that** at least a part of the training data samples of the training data comprises additional non-image input data additionally used for training.

16. Method according to claim 15, **characterized in that** the additional non-image input data comprises tumor biology information and/or tumor therapy information and/or laboratory values and/or subject patient demographics information.

17. A method for tumor lesion characterization, in particular for lung nodule characterization or for characterization and/or tumor biology visualization and assessment of colorectal cancer, comprising the steps of:
- receiving subject patient image data (26) of a region of the anatomy of subject patient,
- analysing said subject patient image data (26) using a trained deep learning algorithm (1),
- determining an output information (2) regarding a tumor lesion based on the step of analysing,
wherein said trained deep learning algorithm (1) has been trained with training data comprising synthetic image data (4) in accordance with any of the methods of claims 7 to 16.

18. Method according to claim 17, **characterized in that** an output information-specific visualization information regarding input regions (22) predictive for specific outcome estimates is determined and the visualization information is output with the output information (2).

19. A system (25) for tumor lesion characterization, in particular for lung nodule characterization or for characterization and/or tumor biology visualization and assessment of colorectal cancer, comprising
- an interface (27) to receive subject patient image data (26) of a region of the anatomy of subject patient,
- an analysing unit (28) for analysing said subject patient image data (26) using a trained deep learning algorithm (1), wherein said trained deep learning algorithm (1) has been trained with training data comprising synthetic image data (4) in accordance with any of the methods of claims 7 to 16,
- a determining unit (29) for determining an output information (2) regarding a tumor lesion based on the step of analysing,
- an interface (30) for outputting said determined output information (2).

20. Computer program, which performs the steps of a method according to any of the claims 1 to 18 if the computer program is executed on a computer.

21. Electronically readable storage medium, on which a computer program according to claim 10 is stored.
